# EUROPEAN PATENT APPLICATION

(11) **EP 4 682 904 A1**
(43) Date of publication of application: **21.01.2026**
(21) Application number: 24382761.5
(22) Date of filing: 15.07.2024
(51) Int. Cl.: G16H 50/30

(54) **COMPUTER SYSTEM AND METHOD FOR GENERATING AN OUTPUT BASED ON MEDICAL DATA**

(71) Applicant: Fresenius Medical Care Deutschland GmbH, 61352 Bad Homburg (DE)
(72) Inventor: Casaña-Eslava, Raul, 61352 Bad Homburg (DE); Silvestre-Llopís, Jordi, 61352 Bad Homburg (DE); Llanes-Jurado, José, 61352 Bad Homburg (DE); Vives-del-Sol, Marcos, 61352 Bad Homburg (DE)
(74) Representative: Wallinger Ricker Schlotter Tostmann

(57) **Abstract**

A computer-implemented method comprises:
evaluating a received signal to be indicative of a medical situation of a person;
then acquiring input data during the medical situation;
preprocessing the input data to obtain preprocessed input data being configured as a subsequent input of a large language model;
extracting medical data representing information on the person and at least one of a medical diagnosis of the person, a medical therapy of the person and a medical condition of the person from the preprocessed input data using the large language model in accordance with an extraction task; and
generating at least one output based on the extracted medical data using the large language model.

## Description

The present invention relates to a computer system and a method for generating an output based on medical data. Moreover, the present invention relates to an administrative system, which comprises the computer system and a patient management system. In addition, the present invention relates to a multi-agent system, which comprises at least two software agents which are configured to communicate with each other and with at least one of the computer system and the administrative system. In general, the present invention relates to the field of medical documentation.

In the case of medical interactions, the documentation of patient discussions before, during and after treatment as well as the documentation of medical events during treatment or data relating to the administration of any medication is a regulatory and medical obligation. This usually requires a considerable amount of time. In the prior art, systems are already known that record spoken language and automatically convert it into written form to reduce the burden of documentation processes.

Furthermore, large language models (LLMs), e.g., generative pretrained transformers (GPT), are known in the prior art, which can process user input and generate an output based on previously used training data. LLMs can thus be used, e.g., to process, reduce or extract data from a given input. The coupling of a system for translating spoken language into text form and the further processing of the text based on an LLM is also known.

An object of the present invention is to provide an improved method and an improved system for facilitating medical documentation.

The object is achieved by the subject matter of the independent claims. Further preferred embodiments of the invention are subject of the dependent claims.

According to a first aspect of the present invention, a computer-implemented method comprises:
evaluating a received signal in terms of whether it is indicative of a medical situation of a person;
when the evaluation yields that the received signal is indicative of a medical situation of the person (10), acquiring input data during the medical situation;
preprocessing the input data to obtain preprocessed input data being configured as a subsequent input of a large language model;
extracting medical data representing information on the person and at least one of a medical diagnosis of the person, a medical therapy of the person and a medical condition of the person from the preprocessed input data using the large language model in accordance with an extraction task; and
generating at least one output based on the extracted medical data using the large language model.

Hereby, in some embodiments, the medical documentation can be facilitated, since the at least one output is automatically generated based on the extracted medical data so that the burden of documentation processes can be further reduced.

In some embodiments, the large language model is a generative pretrained transformer which has been trained to learn statistical relationships from preprocessed input data relating to the medical field and to generate one or more respective outputs depending on a plurality of specific extraction tasks.

In some embodiments, generating the at least one output comprises storing respective separate parts of the extracted medical data, which belong to a respective specific category of the medical data, in separate files having a suitable data format for further documentation and processing.

In the following, preferred embodiments of the computer-implemented method and its further developments are described, which, as far as this is not expressly excluded, can be combined, as desired, with each other and with the other aspects of the invention, which are described below.

In some embodiments, evaluating the received signal comprises checking whether the signal is sent by a medical device, or by a device of a medical personnel, or by a device of the person, or by a network device of a medical facility, and the evaluation yields that the received signal is indicative of a medical situation of the person, when the signal is sent by a medical device, or by a device of a medical personnel, or by a device of the person, or by a network device of a medical facility; wherein
acquiring the input data comprises receiving audio data and/or video data and/or image data and transforming the audio data and/or video data and/or image data into a text document and preprocessing the input data comprises analysing the text document to obtain the preprocessed input data.

In some embodiments, images of the person, in some embodiments a patient, e.g., a dialysis patient, an access or a fistula of the person or voice recordings before/during/after a dialysis treatment can be received, analysed, and categorized by the large language model. In this way, inflammation at access points, discomfort or other pain or suffering of the person can be automatically read in at an early stage, documented in summary form and forwarded in form of the at least one output to medical personnel in a prioritized manner if necessary.

In some embodiments, acquiring the input data may comprise receiving sensor data from a medical sensor device such as a heart rate monitor or a blood pressure monitor and/or receiving a data file from a memory.

In some embodiments, analysing the text document comprises tokenizing the text document such that respective tokens are assigned to respective syntactic substructures of the text document.

In some embodiments, the computer-implemented method further comprises inserting an additional token and/or replacing a token by another token based on a previously stored insertion and replacement matrix.

In a medical context, typically domain-specific language is used, which can even vary within individual specialist areas. In particular, it is possible, e.g., that the definition or semantic breadth of a term is interpreted differently by different medical professionals due to polysemy. Therefore, automated processing of a text input into a large language module can lead to misinterpretations during the ongoing process, as the contextual context is not sufficiently taken into account by the large language model or is lost.

By inserting an additional token and/or replacing a token by another token based on a previously stored insertion and replacement matrix in which a term having different meanings depending on the context is correlated with a predefined meaning of the different meanings, in some embodiments, a clear contextual relationship between the input data can be created so that the processing of the input data by means of the large language model and the resulting output can be improved.

In some embodiments, the computer-implemented method further comprises acquiring the extraction task in the form of an other text document, tokenizing the other text document such that respective tokens are assigned to respective syntactic substructures of the other text document, checking whether an overall number of the tokens assigned to the syntactic substructures of the text document and the tokens assigned to the syntactic substructures of the other text document is greater than a predetermined number of tokens and transforming the text document and the other text document by means of a reduction operation such that after a further tokenizing of the text document and the other text document the overall number of tokens is smaller than or equal to the predetermined number of tokens, if the overall number of tokens is greater than the predetermined number of tokens.

In some embodiments, the text document and the other text document (also referred to as command prompt) together may represent an input prompt of the large language model, wherein the predetermined number of tokens corresponds to a maximum number of tokens that can be processed by the large language model. In this case, the large language model can process the input prompt after the reduction operation has been performed.

The reduction operation can include various operations so that an input prompt can be processed by the large language model if the input prompt exceeds a predefined token limit, i.e., the sum of the number of tokens of the text document and the other text document exceeds the predetermined number of tokens, of the large language model.

In one embodiment (hereinafter referred to as "parallel reduction operation"), a chunking operation is applied to the input prompt in which the input prompt is chunked into a plurality of sub-input prompts, wherein each of the sub-input prompts has a number of tokens that is less than or equal to the predefined token limit. The respective sub-output prompts can then be combined into a final output prompt.

Alternatively, or additionally, the sub-output prompts or the final output prompt can then be used again as input (or input prompt) if, e.g., further data reduction, extraction, analysis or calculation based on the processed data is desired or required. For example, after using the parallel reduction operation once, it may be determined that the number of tokens of the output prompt is still too high for subsequent use. In this case, the parallel reduction operation or an alternative reduction operation can be applied to the output prompt again. The parallel reduction operation is advantageous in that the process can be parallelized and that a high to very high level of detail in the resulting output prompt can be maintained.

In another embodiment (hereinafter referred to as a "sequential reduction operation"), the reduction operation may include a fragmentation operation and subsequent sequential processing by the large language model of an already processed sub-output prompt, together with a new sub-input prompt. In this case, in a first step, a first sub-input prompt is initially generated within the predetermined token limit from first input data and a first command prompt, with the first sub-input prompt then being processed by the large language model, so that a first sub-output prompt is generated. The first sub-output prompt is then processed by the large language model together with second input data and a second command prompt (which, however, can be identical to the first command prompt), so that a second sub-output prompt is generated. This ensures that the token limit is not exceeded by the sum of the first sub-output prompt, second input data and second command prompt. The second input data differs at least partially from the first input data and thus provides new information for the large language model. The algorithm described above is then successively applied to all of the input data. The advantage of the sequential reduction operation lies in particular in the higher level of abstraction compared to parallel processing, especially in the case of long continuous text, e.g., with a high number of tokens due to an audio and/or image recording of a complete treatment of a patient.

Both, parallel and sequential reduction operations, can also be used in any combination and, depending on the problem and specific requirements, can also be effective. However, depending on the kind of the large language model and the type of input data and the corresponding token length, a reduction operation may also be obsolete.

In some embodiments, generating the at least one output comprises creating a summary and/or a medical report based on the extracted medical data, wherein the computer-implemented method further comprises transmitting the summary and/or the medical report to at least one external device; and/or

generating the at least one output comprises outputting an instruction to at least one other external device to emit a warning and/or an alarm based on the extracted medical data and/or outputting a recommendation for setting the medical device based on the extracted medical data.

Hereby, in some embodiments, an individuum having the external device and/or the other device or being close to the external device and/or the other external device can be informed or warned regarding a condition of the person determined based on the extracted medical data.

In some embodiments, the recommendation may include a note to change an ultrafiltration rate for a next dialysis treatment if is determined based on the extracted medical data that an ultrafiltration rate during a previous or current dialysis treatment has led or does lead to pain/discomfort of the person.

In some embodiments,
A) outputting the instruction to the at least one other external device and/or transmitting the summary and/or the medical report to the at least one external device comprises analysing the extracted medical data using a mathematical model to evaluate a risk level of the person, wherein the recommendation is output depending on the evaluated risk level and the summary and/or the medical report are transmitted depending on the evaluated risk level; and/or
B) the medical data comprise at least one of medical numerical values, in some embodiments physiological data of the person, regarding a duration and/or dose and/or scope of the medical therapy, and general anamnesis data of the person, and generating the at least one output comprises creating or changing a medical therapy plan for the person and/or predicting a course of a disease of the person based on the extracted medical data using an other mathematical model for analysing the medical numerical values.

In some embodiments, generating the at least one output comprises at least one of identifying and outputting a time-critical task, identifying and outputting a type and a severity of a disease of the person, and identifying and outputting an urgency of a treatment of the person.

According to a second aspect of the present invention, a computer system is configured to carry out the computer-implemented method described above.

According to a third aspect of the present invention, an administrative system comprises the computer system described above and a patient management system, wherein
the computer system and the patient management system are configured to communicate with each other; and
the patient management system comprises at least one medical device configured to carry out a medical treatment of the person and at least one sensor device configured to capture the audio data and/or the video data and/or the image data.

Here, a communication device of the patient management system may transmit sensor data obtained by the at least one sensor device to a communication unit of the computer system.

In some embodiments, the at least one sensor device is part of or in communication with the at least one medical device and/or the device of the person and/or the device of the medical personnel.

In some embodiments, the at least one medical device is configured to carry out a dialysis treatment of the person.

In some embodiments, the input data relate to and/or represent a communication between the person and a physician or between physicians,
the at least one output comprises clinically important information, in some embodiments allergies of the person and/or important pre-existing conditions of the person; and
the patient management system is configured to generate a medical appointment (between the physician and the person/patient) in accordance with a corresponding output, generated based on the input data using the large language model, from the computer system and to support the physician (e.g., during the medical appointment) based on the clinically important information by automatically hiding certain active ingredients or medical procedures on a display device or outputting a corresponding warning on the display device if the physician suggests (e.g., during the medical appointment) to the person a medical therapy strategy that is critical for the person or enters it in a computer.

Hereby, in some embodiments, damage to the person/patient can be prevented or at least reduced.

In some embodiments, the patient management system is configured to automatically acquire a result of an examination of the person (e.g., from an internal or external data base); and/or
the patient management system is configured to create the medical therapy plan for the person based on the identified urgency of the treatment of the person and/or based on the clinically important information and/or based on the information on the person, in particular the result of the examination of the person; and/or
the administrative system is configured to carry out the identified time-critical task and/or to create an operation plan for the person based on the identified type and severity of the disease of the person.

Hereby, in some embodiments, damage to the person/patient can be prevented or at least reduced. For example, time-critical tasks extracted from input data relating to conversations between physicians can be processed and carried out directly (e.g., inquiries from physicians to a blood bank or questions regarding patient history).

According to a fourth aspect of the present invention, a multi-agent system comprises at least two software agents which are configured to communicate with each other and with at least one of the computer system described above and the administrative system described above, and to receive the at least one output generated by the computer system, wherein
the at least two software agents each comprise an individual large language model configured to process the at least one output and to thereby generate a respective individual software agent output; and
the at least two software agents are configured to interchange the respective individual software agent outputs with each other and to process them in order to jointly create an optimized medical therapy plan for the person based on the processed respective individual software agent outputs.

In some embodiments, the output (data) or the output prompt generated after processing by the computer system described above is read in and processed by a large number of (other) software agents. Here, in the sense of an "expert discussion", different large language models can create an optimized therapy plan or strategy for the person/patient. In some embodiments, the multi-agent system is advantageous in that medical, ethical, resource-related and clinical dimensions can be weighed against each other and even justified by the multi-agent system, wherein the resulting optimized medical therapy plan can then be sent to the physicist and/or medical personnel and/or automatically incorporated directly into the patient management system.

Further embodiments and other objects, advantages and features of the present invention will become apparent from the following detailed description of the invention, the illustration of particular embodiments and the accompanying figures, in which:
- Fig. 1: shows a schematic view of an administrative system according to an embodiment which comprises a computer system according to an embodiment and a patient management system;
- Fig. 2: shows a multi-agent system according to an embodiment; and
- Fig. 3: shows a flow chart for illustrating a computer-implemented method according to an embodiment.

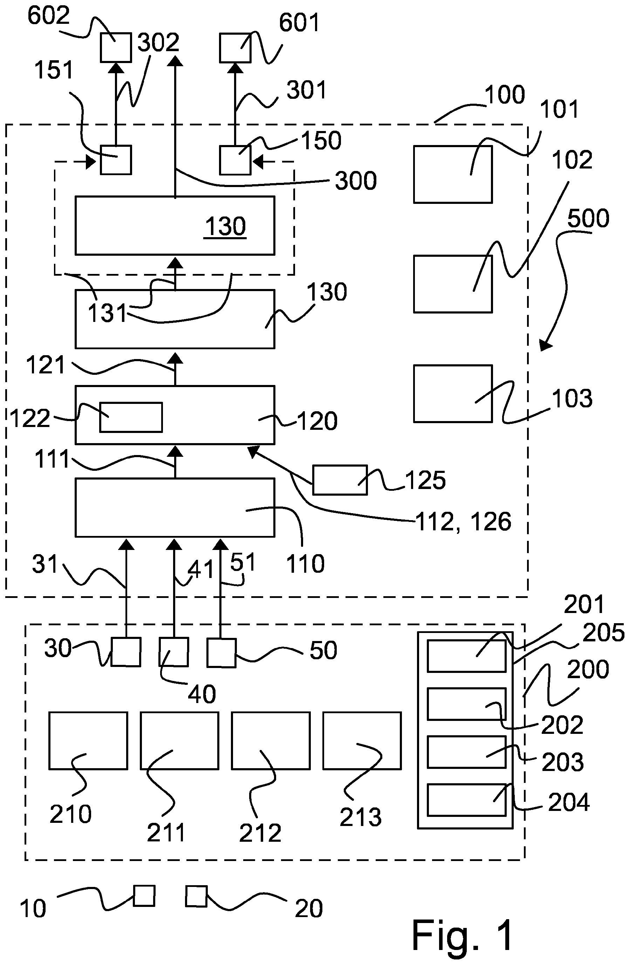

Fig. 1 shows a schematic view of an administrative system 500 according to an embodiment which comprises a computer system 100 according to an embodiment and a patient management system 200.

The computer system 100 is configured to carry out the computer-implemented method according to some embodiments. The computer system 100 comprises a control unit 101, a communication unit 102 and a memory unit 103, wherein the control unit 101 is configured to control the communication unit 102 and the memory unit 103. The patient management system 200 comprises a computer 205 comprising a control device 201, a communication device 202, a memory device 203 and a display device 204, wherein the control device 201 is configured to control the communication device 202, the memory device 203 and the display device 204. The computer system 100 and the patient management system 200 are configured to communicate with each other by means of the communication unit 102 and the communication device 202, respectively.

The communication unit 102 is configured to receive a signal emitted from a medical device 210, or by a device 211 of a medical personnel, or by a device 212 of a person 10, e.g., a patient, or by a network device 213 of a medical facility. In some cases, the medical device 210 and/or the device 211 of the medical personnel and/or the device 212 of the person 10 and/or the network device 213 of the medical facility may be a part of the patient management system 200.

The control unit 101 is configured to evaluate the received signal in terms of whether the signal is indicative of a medical situation of the person 10 or not, in some cases by checking whether the signal is sent by the medical device 210, or by the device 211 of the medical personnel, or by the device 212 of the person 10, or by the network device 213 of the medical facility, wherein the medical device 210 is configured to carry out a medical treatment of the person 10, in some cases a dialysis treatment of the person 10.

In case that the received signal is evaluated to be indicative of the medical situation of the person 10, the communication unit 102 is configured to acquire input data 31, 41, 51 during the medical situation. Here, acquiring the input data 31, 41, 51 comprises receiving audio data 31 and/or video data 41 and/or image data 51 from at least one sensor device 30, 40, 50, which may be part of or in communication with the medical device 210 and/or the device 212 of the person 10 and/or the device 211 of the medical personnel. In same cases, the audio data 31 may be recorded by a microphone 30 of the patient management system 200, the video data 41 may be recorded by a video camera 40 of the patient management system 200 and the image data 51 may be recorded by a camera 50 of the patient management system 200.

In some cases, acquiring the input data may comprise receiving sensor data from a not shown medical sensor device of the patient management system 200 such as a heart rate monitor or a blood pressure monitor and/or receiving a data file from a not shown memory.

Furthermore, acquiring the input data 31, 41, 51 comprises transforming the audio data and/or video data and/or image data and/or sensor data and/or the data file into a text document 111 by means of a data transcriber 110, which may be a software model stored in the memory unit 103 of the computer system 100. The corresponding voice-to-text, video to text and image to text transcriptions can be achieved using various known artificial intelligence (Al) models.

The data transcriber 110 is configured to send the text document 111 to a tokenizer 120, which may be a software module stored in the memory unit 103 of the computer system 100. The tokenizer 120 is configured to analyse the text document 111, wherein analysing the text document 111 may be a part of a preprocessing process of the input data 31, 41, 51 to obtain preprocessed input data 121 being configured as a subsequent input of a large language model (LLM) 130, which may be a software module stored in the memory unit 103 of the computer system 100.

In particular, the tokenizer 120 is configured to tokenize the text document 111 such that respective tokens are assigned to respective syntactic substructures of the text document 111. The substructures of the text document 111 may comprise a series of characters and/or syllables and/or punctuation marks of the text document 111.

In addition, the tokenizer 120 may be configured to insert an additional token and/or replace a token by another token based on an insertion and replacement matrix 122, which may be stored in the memory unit 103 of the computer system 100.

The computer system 100 further comprises an input and output unit 125, e.g., in the form of a touch screen or an interface, by which a user or another computer system can enter a (command) prompt, for example in form of an other text document 112, as an input into the large language model 130 which defines an extraction task 126 to be executed by the large language model 130. Here, the text document 111 and the other text document 112 together represent an input prompt of the large language model 130.

The tokenizer 120 is further configured to tokenize the other text document 112 such that respective tokens are assigned to respective syntactic substructures of the other text document 112. The control unit 101 is configured to check whether an overall number of the tokens assigned to the syntactic substructures of the text document 111 and the tokens assigned to the syntactic substructures of the other text document 112 is greater than a predetermined number of tokens.

In addition, the control unit 101 is configured to transform the text document 111 and the other text document 112 by means of a reduction operation such that after a further tokenizing of the text document 111 and the other text document 112 by means oft the tokenizer 120 the overall number of tokens is smaller than or equal to the predetermined number of tokens, if the overall number of tokens is greater than the predetermined number of tokens.

The large language model 130 is configured to extract medical data 131 representing information on the person 10 and at least one of a medical diagnosis of the person 10, a medical therapy of the person 10 and a medical condition of the person 10 from the preprocessed input data 121 in accordance with a correspondingly formulated extraction task 126.

Furthermore, the large language model 130 is configured to generate at least one output 300, 301, 302 based on the extracted medical data 131.

The reduction operation described above can include various operations so that an input prompt can be processed by the large language model 130 if the input prompt exceeds a predefined token limit, i.e., the sum of the number of tokens of the text document 111 and the other text document 112 exceeds the predetermined number of tokens, of the large language model 130.

In one embodiment (hereinafter referred to as "parallel reduction operation"), a chunking operation is applied to the input prompt in which the input prompt is chunked into a plurality of sub-input prompts, wherein each of the sub-input prompts has a number of tokens that is less than or equal to the predefined token limit. The respective sub-output prompts can then be combined into a final output prompt.

Alternatively, or additionally, the sub-output prompts or the final output prompt can then be used again as input (or input prompt) if, e.g., further data reduction, extraction, analysis or calculation based on the processed data is desired or required. For example, after using the parallel reduction operation once, it may be determined that the number of tokens of the output prompt is still too high for subsequent use. In this case, the parallel reduction operation or an alternative reduction operation can be applied to the output prompt again. The advantage of the parallel reduction operation is, on the one hand, the possibility of parallelizing the process and maintaining a high to very high level of detail in the resulting output prompt.

In another embodiment (hereinafter referred to as a "sequential reduction operation"), the reduction operation may include a fragmentation operation and subsequent sequential processing by the large language model 130 of an already processed sub-output prompt, together with a new sub-input prompt. In this case, in a first step, a first sub-input prompt is initially generated within the predetermined token limit from first input data and a first command prompt, with the first sub-input prompt then being processed by the large language model 130, so that a first sub-output prompt is generated. The first sub-output prompt is then processed by the large language model 130 together with second input data and a second command prompt (which, however, can be identical to the first command prompt), so that a second sub-output prompt is generated. This ensures that the token limit is not exceeded by the sum of the first sub-output prompt, second input data and second command prompt. The second input data differs at least partially from the first input data and thus provides new information for the large language model 130. The algorithm described above is then successively applied to all of the input data. The advantage of the sequential reduction operation lies in particular in the higher level of abstraction compared to parallel processing, especially in the case of long continuous text, e.g., with a high number of tokens due to an audio and/or image recording of a complete treatment of a patient.

Both, parallel and sequential reduction operations, can also be used in any combination and, depending on the problem and specific requirements, can also be effective. However, depending on the kind of the large language model 130 and the type of input data and its token length, a reduction operation may also be obsolete.

In accordance with a corresponding extraction task 126, the large language model 130 may be configured to create a summary 301 and/or a medical report 301 as the at least one output 300, 301, 302 based on the extracted medical data 131. The summary 301 and/or the medical report 301 may then be transmitted by means of the communication unit 102 to at least one external device 601 such as a smartphone, a computer, augmented reality glasses or virtual reality glasses of the medical personnel, in a particular of a physician 20.

Furthermore, in accordance with a corresponding extraction task 126, the large language model 130 may be configured to output an instruction to at least one other external device 602 to emit a warning and/or an alarm based on the extracted medical data 131 and/or to output a recommendation for setting the medical device 210 based on the extracted medical data 131, wherein the communication unit 102 is configured to transmit the respective output 302 to the at least one other external device 602.

In the embodiment shown in Fig. 1, the computer system 100 further comprises a mathematical model 150, which is a software module which may be stored in the memory unit 103, and which is configured to analyse the extracted medical data 131 in order to evaluate a risk level of the person 10, wherein the communication unit 102 is configured to output the recommendation depending on the evaluated risk level and to output the summary 301 and/or the medical report 301 depending on the evaluated risk level.

In some embodiments, the medical data 131 comprise at least one of medical numerical values, in particular physiological data of the person 10, regarding a duration and/or dose and/or scope of the medical therapy, and general anamnesis data of the person 10. In this case, in the embodiment shown in Fig. 1, the computer system 100 may further comprise an other mathematical model 151, which is a software module which may be stored in the memory unit 103. The other mathematical model 151 is configured to analyse the medical numerical values and to create or change a medical therapy plan for the person 10 and/or to predict a course of a disease of the person 10 based on the analysis of the medical numerical values. Here, the communication unit 102 is configured to output the created or changed medical therapy plan for the person 10 and/or the predicted course of the disease of the person 10 to the other external device 602.

In addition, in accordance with a corresponding extraction task 126, the large language model 130 may be configured to identify and output a time-critical task and/or to identify and output a type and a severity of a disease of the person 10 and/or to identify and output an urgency of a treatment of the person 10, based on the extracted medical data 131.

In some embodiments, the input data 31, 41, 51 relate to a communication between the person 10 and the physician 20. In this case, the at least one output 300, 301, 302 may comprise clinically important information, in particular allergies of the person 10 and/or important pre-existing conditions of the person 10, wherein the patient management system 200 is configured to generate a medical appointment (between the physicist and the person) in accordance with a corresponding output 300, generated based on the input data 31, 41, 51 using the large language model, from the computer system 100 and to support the physician 20 based on the clinically important information by automatically hiding certain active ingredients or medical procedures on the display device 204 or outputting a corresponding warning on the display device 204 if the physician 20 suggests to the person 10 a medical therapy strategy that is critical for the person 10 or enters it in the computer 205.

Here, the patient management system 200 may be configured to automatically acquire a result of an examination of the person 10. Furthermore, the patient management system 200 may be configured to create the medical therapy plan for the person 10 based on the identified urgency of the treatment of the person 10 and/or based on the clinically important information and/or based on the information on the person 10, in particular the result of the examination of the person 10. In addition, the administrative system 500 may be configured to carry out the identified time-critical task and/or to create an operation plan for the person 10 based on the identified type and severity of the disease of the person 10.

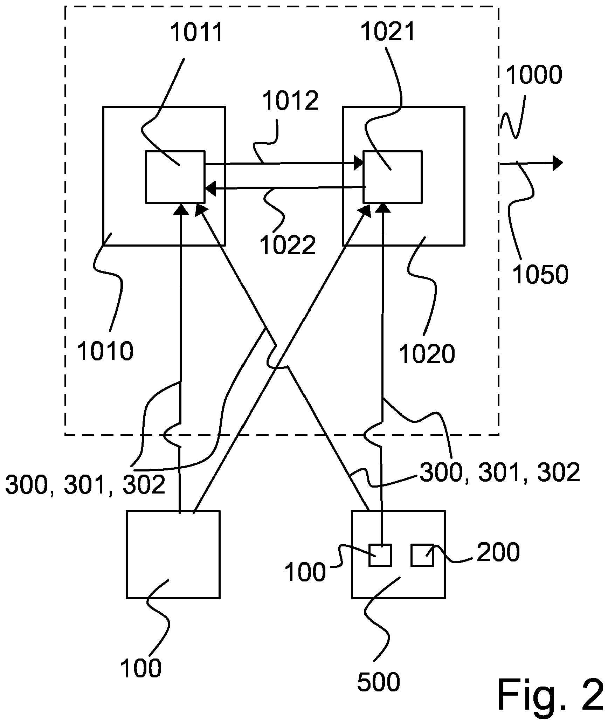

Fig. 2 shows a multi-agent system 1000 according to an embodiment. The multi-agent system 1000 comprises at least two software agents 1010, 1020, in the example shown in Fig. 2, a software agent 1010 and an other software agent 1020. The software agent 1010 and the other software agent 1020 are configured to communicate with each other and with at least one of the computer system 100 and the administrative system 500. Furthermore, each of the software agent 1010 and the other software agent 1020 is configured to receive the at least one output 300, 301, 302 generated by the computer system 100. The software agent 1010 comprises an individual large language model 1011 and the other software agent 1020 comprises an other individual large language model 1021.

The individual large language model 1011 is configured to process the at least one output 300, 301, 302 and to thereby generate an individual software agent output 1012, and the other individual large language model 1021 is configured to process the at least one output 300, 301, 302 and to thereby generate an other individual software agent output 1022.

The software agent 1010 and the other software agent 1020 are configured to interchange the individual software agent output 1012 and the other individual software agent output 1022, respectively, with each other and to process the respective received individual software agent output 1012, 1022 in order to jointly create an optimized medical therapy plan 1050 for the person 10 based on the processed respective individual software agent outputs 1012, 1022.

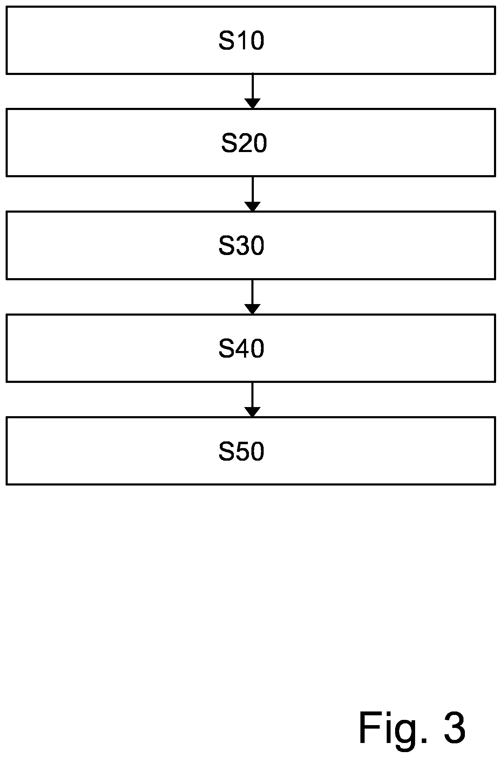

Fig. 3 shows a flow chart for illustrating a computer-implemented method according to an embodiment.

In a step S10 of the computer-implemented method, a received signal is evaluated to be indicative of a medical situation of a person 10.

Here, evaluating the received signal may comprise checking whether the signal is sent by a medical device 210, or by a device 211 of a medical personnel, or by a device 212 of the person, or by a network device 213 of a medical facility.

In a step S20 of the computer-implemented method, which is carried out after step S10, input data 31, 41, 51 are acquired during the medical situation.

Here, acquiring the input data 31, 41, 51 may comprise capturing audio data and/or video data and/or image data and transforming the audio data and/or video data and/or image data into a text document 111.

In a step S30 of the computer-implemented method, the input data 31, 41, 51 are preprocessed to obtain preprocessed input data 121 being configured as a subsequent input of a large language model 130.

Here, preprocessing the input data 31, 41, 51 may comprise analysing the text document 111 to obtain the preprocessed input data 121, wherein analysing the text document 111 may comprise tokenizing the text document 111 such that respective tokens are assigned to respective syntactic substructures of the text document 111.

In some embodiments, the computer-implemented method may further comprise inserting an additional token and/or replacing a token by another token based on a previously stored insertion and replacement matrix 122.

Alternatively or in addition, the computer-implemented method may further comprise acquiring the extraction task 126 in the form of an other text document 112, tokenizing the other text document 112 such that respective tokens are assigned to respective syntactic substructures of the other text document 112, checking whether an overall number of the tokens assigned to the syntactic substructures of the text document 111 and the tokens assigned to the syntactic substructures of the other text document 112 is greater than a predetermined number of tokens and transforming the text document 111 and the other text document 112 by means of a reduction operation such that after a further tokenizing of the text document 111 and the other text document 112 the overall number of tokens is smaller than or equal to the predetermined number of tokens, if the overall number of tokens is greater than the predetermined number of tokens.

In a step S40 of the computer-implemented method, medical data 131 representing information on the person 10 and at least one of a medical diagnosis of the person 10, a medical therapy of the person 10 and a medical condition of the person 10 are extracted from the preprocessed input data 121 using the large language model 130 in accordance with an extraction task 126.

In a step S50 of the computer-implemented method, at least one output 300, 301, 302 is generated based on the extracted medical data 131 using the large language model 130.

In some embodiments, generating the at least one output 300, 301, 302 may comprise creating a summary 301 and/or a medical report 301 based on the extracted medical data 131. In this case the method may further comprise transmitting the summary and/or the medical report to at least one external device 601.

Alternatively, or in addition, generating the at least one output 300, 301, 302 may comprise outputting an instruction 302 to at least one other external device 602 to emit a warning and/or an alarm based on the extracted medical data 131 and/or outputting a recommendation for setting the medical device 210 based on the extracted medical data 131.

Outputting the instruction 302 to the at least one other external device 602 and/or transmitting the summary and/or the medical report to the at least one external device 601 may comprise analysing the extracted medical data 131 using a mathematical model 150 to evaluate a risk level of the person 10. In this case, the recommendation may be output depending on the evaluated risk level, and the summary 301 and/or the medical report 301 may be transmitted depending on the evaluated risk level.

Alternatively, or in addition, the medical data 131 may comprise at least one of medical numerical values, in some embodiments physiological data of the person 10, regarding a duration and/or dose and/or scope of the medical therapy, and general anamnesis data of the person 10. In this case, generating the at least one output 302 may comprise creating or changing a medical therapy plan for the person 10 and/or predicting a course of a disease of the person 10 based on the extracted medical data 131 using an other mathematical model 151 for analysing the medical numerical values.

In some embodiments, generating the at least one output 300, 301, 302 comprises at least one of identifying and outputting a time-critical task, identifying and outputting a type and a severity of a disease of the person 10, and identifying and outputting an urgency of a treatment of the person 10.

### LIST OF REFERENCE SIGNS

- 10: person
- 20: physician
- 30, 40, 50: sensors
- 31, 41, 51: input data
- 100: computer system
- 101: control unit
- 102: communication unit
- 103: memory unit
- 110: data transcriber
- 111: text document
- 112: other text document
- 120: tokenizer
- 121: preprocessed input data
- 122: insertion and replacement matrix
- 125: input and output unit
- 126: extraction task
- 130: large language model (LLM)
- 131: medical data
- 150: mathematical model
- 151: other mathematical model
- 200: patient management system
- 201: control device
- 202: communication device
- 203: memory device
- 204: display device
- 205: computer
- 210: medical device
- 211: device of a medical personnel
- 212: device of the person
- 213: network device of a medical facility
- 300: output
- 301: output
- 302: output
- 500: administrative system
- 601: external device
- 602: other external device
- 1000: multi-agent system
- 1010: software agent
- 1011: individual large language model
- 1012: individual software agent output
- 1020: other software agent
- 1021: other individual large language model
- 1022: other individual software agent output
- 1050: optimized medical therapy plan

## Claims

1. A computer-implemented method, comprising:
evaluating a received signal in terms of whether it is indicative of a medical situation of a person (10);
when the evaluation yields that the received signal is indicative of a medical situation of the person (10), acquiring input data (31, 41, 51) during the medical situation;
preprocessing the input data (31, 41, 51) to obtain preprocessed input data (121) being configured as a subsequent input of a large language model (130);
extracting medical data (131) representing information on the person (10) and at least one of a medical diagnosis of the person (10), a medical therapy of the person (10) and
a medical condition of the person (10) from the preprocessed input data (121) using the large language model (130) in accordance with an extraction task (126); and
generating at least one output (300, 301, 302) based on the extracted medical data (131) using the large language model (130).

2. The computer-implemented method according to claim 1, wherein evaluating the received signal comprises checking whether the signal is sent by a medical device (210), or by a device (211) of a medical personnel, or by a device (212) of the person (10), or by a network device (213) of a medical facility, and the evaluation yields that the received signal is indicative of a medical situation of the person (10), when the signal is sent by a medical device (210), or by a device (211) of a medical personnel, or by a device (212) of the person (10), or by a network device (213) of a medical facility; and
wherein acquiring the input data (31, 41, 51) comprises receiving audio data and/or video data and/or image data and transforming the audio data and/or video data and/or image data into a text document (111) and preprocessing the input data (31, 41, 51) comprises analysing the text document (111) to obtain the preprocessed input data (121).

3. The computer-implemented method according to the preceding claim, wherein analysing the text document (111) comprises tokenizing the text document (111) such that respective tokens are assigned to respective syntactic substructures of the text document (111).

4. The computer-implemented method according to claim 3, further comprising inserting an additional token and/or replacing a token by another token based on a previously stored insertion and replacement matrix (122).

5. The computer-implemented method according to claim 3 or 4, further comprising acquiring the extraction task (126) in the form of an other text document (112), tokenizing the other text document (112) such that respective tokens are assigned to respective syntactic substructures of the other text document (112), checking whether an overall number of the tokens assigned to the syntactic substructures of the text document (111) and the tokens assigned to the syntactic substructures of the other text document (112) is greater than a predetermined number of tokens and transforming the text document (111) and the other text document (112) by means of a reduction operation such that after a further tokenizing of the text document (111) and the other text document (112) the overall number of tokens is smaller than or equal to the predetermined number of tokens, if the overall number of tokens is greater than the predetermined number of tokens.

6. The computer-implemented method according to any one of the claims 2 to 5, wherein generating the at least one output (301) comprises creating a summary and/or a medical report based on the extracted medical data (131) and the method further comprises transmitting the summary and/or the medical report to at least one external device (601); and/or
wherein generating the at least one output (301) comprises outputting an instruction to at least one other external device (602) to emit a warning and/or an alarm based on the extracted medical data (131) and/or outputting a recommendation for setting the medical device (210) based on the extracted medical data (131).

7. The computer-implemented method according to claim 6, wherein
A) outputting the instruction to the at least one other external device and/or transmitting the summary and/or the medical report to the at least one external device (601) comprises analysing the extracted medical data (131) using a mathematical model (150) to evaluate a risk level of the person (10), and wherein the recommendation is output depending on the evaluated risk level and the summary and/or the medical report are transmitted depending on the evaluated risk level; and/or
B) the medical data (131) comprise at least one of medical numerical values, in particular physiological data of the person (10), regarding a duration and/or dose and/or scope of the medical therapy, and general anamnesis data of the person (10), and wherein generating the at least one output (301) comprises creating or changing a medical therapy plan for the person (10) and/or predicting a course of a disease of the person (10) based on the extracted medical data (131) using an other mathematical model (151) for analysing the medical numerical values.

8. The computer-implemented method according to any one of the preceding claims, wherein generating the at least one output (300, 301, 302) comprises at least one of identifying and outputting a time-critical task, identifying and outputting a type and a severity of a disease of the person (10), and identifying and outputting an urgency of a treatment of the person (10).

9. A computer system (100), configured to carry out the computer-implemented method according to any one of the preceding claims.

10. An administrative system (500), comprising the computer system (100) according to claim 9 and a patient management system (200), wherein
the computer system (100) and the patient management system (200) are configured to communicate with each other; and
the computer system (100) is configured to carry out the computer-implemented method according to claim 2 and the patient management system (200) comprises at least one medical device (210) configured to carry out a medical treatment of the person (10) and at least one sensor device (30, 40, 50) configured to capture the audio data and/or the video data and/or the image data.

11. The administrative system (500) according to the preceding claim, wherein the at least one sensor device (30, 40, 50) is part of or in communication with the at least one medical device (210) and/or the device (212) of the person (10) and/or the device (211) of the medical personnel.

12. The administrative system (500) according to any one of claims 10 and 11, wherein the at least one medical device (210) is configured to carry out a dialysis treatment of the person (10).

13. The administrative system (500) according to any one of claims 10 to 12, wherein
the input data (31, 41, 51) relate to a communication between the person (10) and a physician (20);
the at least one output (300, 301, 302) comprises clinically important information, in particular allergies of the person (10) and/or important pre-existing conditions of the person (10), and
the patient management system (200) is configured to generate a medical appointment in accordance with a corresponding output (300), generated based on the input data (31, 41, 51) using the large language model (130), from the computer system (100) and to support the physician (20) based on the clinically important information by automatically hiding certain active ingredients or medical procedures on a display device (204) or outputting a corresponding warning on the display device (204) if the physician (20) suggests to the person (10) a medical therapy strategy that is critical for the person (10) or enters it in a computer (205).

14. The administrative system (500) according to claim 13, wherein
the patient management system (200) is configured to automatically acquire a result of an examination of the person (10); and/or
the computer system (100) is configured to carry out the computer-implemented method according to claims 7 and 8, and the patient management system (200) is configured to create the medical therapy plan for the person (10) based on the identified urgency of the treatment of the person (10) and/or based on the clinically important information and/or based on the information on the person (10), in particular the result of the examination of the person (10); and/or
the computer system (100) is configured to carry out the computer-implemented method according to claim 8, and the administrative system (500) is configured to carry out the identified time-critical task and/or to create an operation plan for the person (10) based on the identified type and severity of the disease of the person (10).

15. A multi-agent system (1000), comprising at least two software agents (1010, 1020) which are configured to communicate with each other and with at least one of the computer system (100) according to claim 9 and the administrative system (500) according to any one of claims 10 to 14, and to receive the at least one output (300, 301, 302) generated by the computer system (100), wherein
the at least two software agents (1010, 1020) each comprise an individual large language model (1011, 1021) configured to process the at least one output (300, 301, 302) and to thereby generate a respective individual software agent output (1012, 1022); and
the at least two software agents (1010, 1020) are configured to interchange the respective individual software agent outputs (1012, 1022) with each other and to process them in order to jointly create an optimized medical therapy plan (1050) for the person (10) based on the processed respective individual software agent outputs (1012, 1022).
